# EUROPEAN PATENT SPECIFICATION

(11) **EP 1 465 550 B1**
(45) Date of publication and mention of the grant of the patent: **23.03.2016**
(21) Application number: 02784919.9
(22) Date of filing: 19.12.2002
(51) Int. Cl.: A61F 2/06, A61F 2/07, A61F 2/95

(54) **A DEVICE FOR USE IN INTRALUMINAL GRAFTING**
GERÄT ZUR VERWENDUNG BEIM INTRALUMINALEN GRAFTING
DISPOSITIF UTILISE DANS LES GREFFES ENDOLUMINALES

(30) Priority: 20.12.2001 AU PR969201
(43) Date of publication of application: 13.10.2004
(73) Proprietor: W.L. Gore & Associates, Inc., Newark DE 19714 (US)
(72) Inventor: White, Geoffrey H., Birchgrove, NSW 2041 (AU)
(74) Representative: Wilson, Gary
(86) International application number: PCT/AU2002/001725
(87) International publication number: WO 2003/053283

(56) References cited:
- WO-A-01/21108
- WO-A-01/66038
- WO-A1-97/09008
- WO-A1-97/29716
- WO-A1-98/27895
- WO-A2-01/41678
- WO-A2-01/66038
- US-A- 5 693 088
- US-A- 6 015 431
- US-B1- 6 228 845

## Description

### Filed of the Invention

The present invention relates to a device for use in intraluminal grafting and particularly to a device which secures an intraluminal graft or stent within a vessel of a patient.

### Background Art

Intraluminal grafts are used in the treatment of several diseases including aneurysmal disease and stenotic disease. The graft is typically used to by-pass a diseased area of a vessel thereby enabling the flow of blood therethrough.

Difficulties often arise in the placement and sealing of such grafts and, further, in maintaining the graft within a vessel of a patient. This may be the case either because the diseased vessel has an atypical configuration or simply because a particular individual has a unique anatomy. For example, the vessel may bulge, form a cone shape or may branch from a first vessel at more of an angle than would be expected.

Conventional intraluminal grafts often do not align or seal well with such a diseased or anatomically unique vessel structure. Accordingly, such ill-fitting grafts have a tendency to slip within the vessel, reducing the effectiveness of the graft and in some cases causing the graft to occlude an opening of a pre-or post-branching vessel.

Such a drawback may stem from the fact that most conventional intraluminal grafts are made from material having properties to optimise blood flow through the graft. These materials, however, may not be particularly suitable for securing and sealing the graft within a vessel. Accordingly, by selecting a material with good blood flow properties, a sacrifice is made in respect of the ability of the material to secure the graft in the vessel.

It has been suggested that conventional intraluminal grafts be coated with polyurethane, a material which may be incorporated into vessel walls. However, such a coating on the outside of an intraluminal graft greatly increases the diameter of the compressed graft. In turn, the graft does not fit within an introducer catheter and is too bulky to be introduced into a vessel of a patient using minimally invasive techniques.

WO 97/09008, which is considered to be the prior art closest to the subject matter of claim 1, discloses a tubular prosthesis which is implanted at a target location within a body lumen by trans-luminal placing and embedding an expansible prosthesis body within a sealing layer. The sealing layer occludes at least a circumferential band within an interface region between the prosthesis body and the inner wall of the body lumen, thus providing for blockage of body lumen flow past the prosthesis. The sealing layer may be introduced prior to or simultaneously with the prosthesis body. A tubular prosthesis may be implanted in blood vessels, particularly to protect aneurysms.

WO98/27895 discloses an endolumenal stent-graft with a leak-resistant seal. WO01/66038 discloses an endovascular device having a stent.

The present invention aims to provide a device for us with an intraluminal graft which provides a means to overcome the abovementioned problems of the prior art.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed in Australia before the priority date of each claim of this application.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

### Summary of the Invention

In a first aspect, the present invention provides an engagement device for an intraluminal graft or stent, the device comprising a main body having (i) an inner wall which defines a receiving region to receive and engage at least a portion of an intraluminal graft or stent and (ii) an outer wall to engage the wall of the vessel of a patient, wherein said main body is capable of expanding or being expanded from a first radially compressed state to a second radially expanded state such that when in its radially expanded state, the outer wall engages the wall of the vessel so that, in use, flow of blood in a vessel to which the engagement device is fitted is channelled through the intraluminal graft or stent thereby preventing leakage of blood into the damaged part of the vessel,

### characterised in that

the engagement device is so arranged that, in use, it is unattached to said intraluminal graft or stent prior to and during delivery of the device to a vessel.

In a second aspect, the present invention provides an intraluminal assembly for placement in a vessel of a patient, the assembly comprising:
(i) an engagement device in accordance with the first aspect of the present invention; and
(ii) an intraluminal graft or stent capable of expanding or being expanded from a first radially compressed configuration to a second radially expanded configuration.

The main body of the device may be substantially cylindrical wherein the entire outer wall of the cylindrical main body engages the wall of a vessel to effectively seal the device of the present invention in said vessel. The device essentially forms a collar or ring around one or both ends of the intraluminal graft or stent. Alternatively, the device may form a collar or ring around a portion of the length of said intraluminal graft or stent, between said ends.

In a further embodiment, the main body of the device is generally frusto-conical in shape. Preferably, the internal wall is substantially straight relative to the longitudinal axis of the main body of the device and the outer wall is angled relative to the longitudinal axis of the main body. The result is that the outer wall forms the frusto-conical structure of the main body.

A generally frusto-conical shape of the main body may further secure the main body of the device within a vessel wall. Further, in this embodiment, because the internal wall is substantially straight relative to the longitudinal axis of the main body, the lumen, which may be defined by the internal wall is also relatively straight and therefore suited to receiving a standard intraluminal graft or stent therein.

It should be noted, however, that the main body of the device may take many shapes and may be particularly configured to confirm with an atypical anatomy of a vessel. Accordingly, in addition to the above examples, it is envisaged that the internal wall of the main body may taper along the length of the main body and, therefore, in the case where the internal wall defines a lumen, the diameter of the lumen may vary along the length of the main body.

The main body of the device typically extends from a first end to a second end wherein either or both ends may be chamfered or angled relative to the longitudinal axis of the main body. This embodiment may be particularly useful in cases where the section of a target vessel to be bridged by an intraluminal graft or stent is close to branching vessels. In this case, it is desirable to position the graft or stent as close to the branch as possible without occluding the opening of the branched vessels. If the branching vessels are not perfectly aligned (as is commonly the case), a flat, rather than an angled end may occlude the opening to one of the branching vessels. Similarly, in the case of an aneurysm which causes the vessel wall to deviate at an angle relative to its original configuration, a main body having an angled end may be suitable for engaging the walls of such a section of the vessel.

The main body of the device may be curved relative to its longitudinal axis or, alternatively form a substantially sinusoidal configuration relative to its longitudinal axis.

The device of the present invention, in addition to comprising a main body may further comprise a skirt member which may be attached to or integral with the first end of the main body and depend therefrom.

The main body of the device may be made from any number of materials including, but not limited to, a polyurethane polymer including polyurethane or polycarbonate. The main body may be a foam or sponge material. Further, different parts or lengths of the main body may be made from different types of material.

Preferably, the material of the main body is selected for its biocompatibility and ability to mould with the anatomy of the vessel in which it is positioned and to integrate with the tissue of the vessel wall. In this way, the main body of the device preferably becomes firmly integrated and secured within the vessel wall.

The main body of the device may be self expandable or expanded by some other means. The expansion ratio of the main body may be variable but is preferably from about a ratio of 1.5:1 up to about 10:1.

The main body may be formed of a material that is compressed or contracted through dehydration. In this case, upon rehydrating the material of the main body, the main body expands.

The main body of the device may further contain within or on its structure, metallic wires or wireforms. The wires may be formed from Nitinol^{™} stainless steel or other alloys such as tantalum or Elgiloy.

In the embodiment wherein the wires are made from a shape memory material, the wires may be positioned within or on the main body such that upon delivery of the main body of the device to a vessel of a patient, the wires take on a "memorised" configuration, that is, typically the main body of the device moves to its expanded configuration and engages the vessel wall. The movement of the wires to the predetermined "memorised" configuration may bring the entire length of the outer wall of the main body into engagement with a vessel wall or alternatively, only a portion of the outer wall of the main body may engage the vessel wall.

Preferably, the cross-sectional diameter of the main body of the device, when it is in its radially compressed state is less than 5mm and, when in its radially expanded state up to approximately 35mm or other suitable diameter to allow the outer wall of the main body to engage a vessel wall.

The region of the main body from the internal wall to the outer wall is typically around 2mm when the main body is in the radially compressed state and around 4mm when the main body is in the radially expanded state, although in the embodiment of the invention wherein the main body is frustoconical in shape said region between the internal wall and the outer wall will vary in thickness along the length of the main body.

The device of the present invention may be used in the grafting or stenting of diseased arteries of a patient including the aorta and the visceral arteries such as the renal and mesenteric arteries, the iliac artery and the sub-clavian artery. It may also be used in the grafting or stenting of the peripheral vasculature, the coronary circulation, the hepato-biliary and genito-urinary tracts.

The main body of the device may be coated with any of a number of agents including agents such as fibrin or fibrin glue which facilitate the incorporation of the device into the vessel wall or ingrowth of the vessel wall into the device. Other agents include but are not limited to heparin, warfarin, ticloidine, dipyramole, GPIIb/IIIa receptor blockers, thromboxane inhibitors, seratonin antagonists, prostanoids, calcium channel blockers, ACE inhibitors, angiopeptin, steroids, non-steroidal antiinflammatory drugs, enzymes, nitric oxide genetic modulators.

Preferably, the main body of the device includes radiopaque markers to facilitate positioning of the device within a vessel of a patient.

During use of the device of the present invention, the main body of the device is initially in its radially compressed state to enable delivery of the device through an introducer catheter. Upon deployment of the device into a selected vessel, the main body may be caused to expand, or may be allowed to self-expand into its radially expanded state. It is envisaged that a device made from a compressible foam or sponge material could be delivered in this manner.

When in its radially compressed state, the main body of the device may be a substantially straight cylinder which is compressed to fit within an introducer catheter. Upon release and adoption of the radially expanded state, in addition to expanding in size, the shape of the main body may change, for example from a substantially straight cylindrical tube to a fiusto-conical tubular member.

As described above, the main body of the device may self expand upon release into a vessel or alternatively, the main body may be expanded by the force of an inflating balloon within the lumen or recess of the main body or by some other mechanically applied force.

Alternatively, the main body of the device may be made from a shape memory material or may include wire or wireforms made from a shape memory material as mentioned above. The patient's body temperature causes the temperature of the main body of the device to change, thereby enabling the main body to self-expand and take on a "memorised" shape.

In a further embodiment, the main body of the device may self expand following deployment of the device from an introducer catheter used to introduce the device of the invention into a vessel of a patient. This particular embodiment relies upon spring expansion of the material of the main body following release of the compressive force of the introducer catheter.

The main body of the device may include one or a number of projections extending from the outer wall of the main body. Such projections may be adapted to engage with and further secure the device to a vessel wall of a patient. The projections may be made from a number of materials including stainless steel or a shape memory alloy such as NitinolTM. In the latter case, upon deployment of the device into a vessel, the change in temperature of the surrounding environment may cause the projections to move from a first position substantially aligned with the main body of the device to a second position wherein the projections extend outwardly and away from the main body of the device and may engage the vessel wall.

The internal wall of the main body may be treated with a material which enhances the engagement or adherence between said internal wall of the main body and an intraluminal graft or stent received within the lumen or recess defined by said internal wall. Alternatively, the internal wall of the main body may have a series of projections which engage the intraluminal graft or stent, securing the said graft or stent to the main body of the device.

The device of the present invention may be custom made for a particular anatomy of a patient. In this regard, the vessel of a patient may be imaged and, using computer modelling, a device of suitable size and dimension prepared to fit within the vessel.

The device of the present invention may or may not be attached to an intraluminal graft or stent prior to and during delivery of the device to a target vessel.

The device of the invention further includes at least one stay or holding catheter releasably connected to a portion of the main body of the device. The stay or holding catheter is preferably flexible but of sufficient rigidity to hold the device in a desired position during deployment of the device within a target vessel and during deployment of an intraluminal graft or stent within said vessel. The stay or catheter may be introduced from downstream of the disease site or alternatively from upstream of the site. For example, if the device of the present invention is used with a graft or stent to bridge a region of an aorta of a patient, the stay or catheter may be introduced through either the femoral or the brachial artery of a patient.

There is also described a method of positioning an engagement device for an intraluminal graft or stent according to the first aspect of the invention in a diseased vessel of a patient, the method including the steps of ; (i) introducing a catheter or other delivery device into a vein, artery or other vessel of a patient until a distal end of said catheter or other delivery device is positioned at or adjacent a target region of the diseased vessel; (ii) causing the engagement device for an intraluminal graft or stent to be carried through the catheter or other delivery device to said target region, with the main body of said device in its radially compressed state; (iii) releasing the main body from the distal end of the catheter or other delivery device and causing or allowing said main body to move to its radially expanded state within the target region of the diseased vessel such that at least part of the main body engages a wall of said target region of the vessel; (iv) positioning a radially compressed intraluminal graft or stent at least partially within the receiving region of the main body; (v) causing or allowing the intraluminal graft or stent to move to a radially expanded state such that at least a portion of the intraluminal graft or stent engages with said receiving region of the main body; and (vi) withdrawing the catheter or other delivery device along with any other apparatus used to introduce the engagement device for an intraluminal graft or stent and/or the intraluminal graft or stent from the patient.

The engagement device for an intraluminal graft or stent may be pre-packaged in the catheter or other delivery device or alternatively, may be passed through said catheter or other delivery device as a separate step.

A number of engagement devices for an intraluminal graft or stent may be delivered to the target region. For example said devices may be concentrically arranged such that the resultant cross-sectional diameter of the devices is increased.
Alternatively, a number of devices may be introduced along the length of the intraluminal graft or stent to secure and seal said graft or stent in a vessel.

The engagement device for an intraluminal graft or stent of the invention may be delivered to the target region in the vessel as a first step, independent of the delivery of the intraluminal graft or stent. In this embodiment, the device may be passed through an introducer catheter to the target region, whereupon it is released and caused or allowed to take on its radially expanded state such that it engages the vessel wall. With the device in an expanded state, an intraluminal graft or stent may be introduced (using the same or a different introducer catheter) such that a portion of the intraluminal graft or stent is received by said receiving region of the main body of the device.

As noted above, the main body of the device may be cylindrical in structure with an internal lumen defined by an internal wall extending therethrough. In this embodiment, a portion of the intraluminal graft or stent may be positioned within the lumen of the main body. It is preferred that the positioning of the intraluminal graft provides an optimal overlap between the intraluminal graft and the device of the invention to thereby secure said graft or stent to the device.

Alternatively, the device together with an intraluminal graft or stent may be packaged together as a single assembly for delivery to a vessel of a patient. In this regard, it is envisaged that the internal wall of the main body of the device is attached to a portion of a wall of the intraluminal graft or stent. Accordingly, the assembly may be carried through the introducer catheter and released within the target region of a vessel. Upon release into the vessel, the main body of the device is caused or allowed to move to its radially expanded state.

In cases where a bifurcated graft is required, it is envisaged that the graft is inserted through one side of the main body eg the right femoral artery and the device of the present invention introduced through the other side eg the left femoral artery.

The intraluminal graft or stent may be caused or allowed to radially expand upon release of the assembly or, alternatively, following the radial expansion of the main body of the device of the present invention.

The radial expansion of the intraluminal graft or stent may further secure the outer wall of the main body of the device to a wall of the vessel, that is, an added force is applied to secure the device within the vessel.

In another aspect, the present invention consists in a sealing member for an intraluminal graft or stent said sealing member comprising a collar or ring member which substantially surrounds the circumference of a portion of said intraluminal graft or stent such that when the intraluminal graft or stent is *in situ* within a vessel of a patient, the sealing member provides a substantially blood tight seal around the intraluminal graft or stent.

In a further aspect, the present invention consists in a method of providing a substantially blood tight seal around an intraluminal graft or stent in a vessel of a patient, said method including the step of positioning a sealing member comprising a collar or ring member substantially around the circumference of a portion of said intraluminal graft or stent.

### Brief Description of the Drawings

Figure 1 is a side elevational view of a device of the present invention in a radially expanded state.
Figures 2a and 2b depict delivery of the device of one embodiment of the invention to a target vessel.
Figure 3 a depicts an example of an atypical anatomy of a section of the aorta of a patient.
Figure 3b shows the placement of the device of the present invention in the section of the aorta depicted in Figure 3 a.

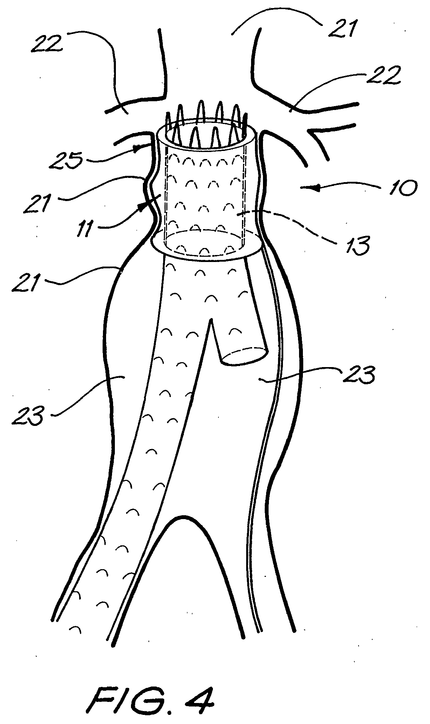

Figure 4 shows the device of the present invention together with an intraluminal graft positioned within the aorta of a patient.
Figures 5a to 5f show various embodiments of the device of the present invention.
Figure 6a shows a further embodiment of the device of the present invention.
Figure 6b shows an application of the embodiment depicted in Figure 6a.
Figure 7a shows another embodiment of the device of the present invention.
Figure 7b depicts an application of the embodiment shown in Figure 7a.
Figure 8a depicts a further embodiment of the device of the present invention.
Figure 8b shows an application of the embodiment of Figure 8a.
Figure 9a shows another embodiment of the device of the present invention.
Figure 9b shows an application of the embodiment depicted in Figure 9a.

### Preferred Mode of Carrying out the Invention

The intraluminal device of the present invention is generally depicted as 10 in the accompanying drawings. The device 10 has a main body 11 with a receiving region 12 adapted to receive part of an intraluminal graft 13 therein.

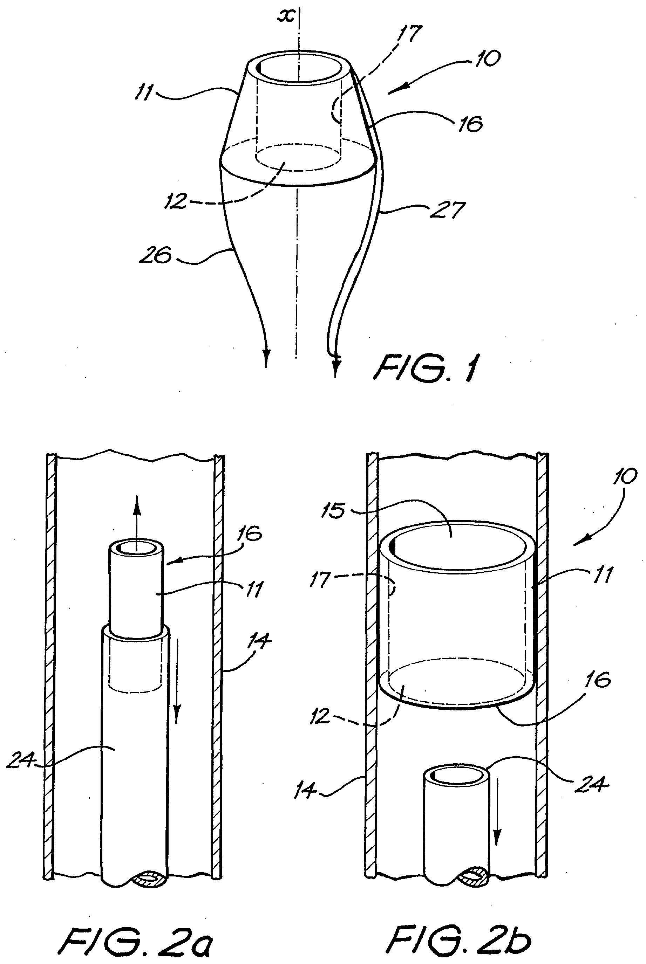

As shown, the main body 11 is capable of expanding or being expanded from a first radially compressed state (shown in Figure 2a) to a second radially expanded state (shown in Figure 2b) such that when in the second radially expanded state, the main body 11 engages a wall of a vessel 14 in which it is positioned.

In each depiction, the main body 11 has an internal lumen 15 extending therethrough. The internal lumen 15 receives the intraluminal graft 13.

In Figures 2a and b, the main body 11 of the device 10 can be seen to be substantially cylindrical.

As shown in Figure 1, the main body 11 of the device 10 may be of a generally frusto-conical configuration. In this embodiment, it can be seen that the main body 11 has an outer wall 16 for engaging a wall of a vessel 14 and an internal wall 17 which defines the lumen 15.

In Figure 1, the internal wall 17 is substantially straight relative to the longitudinal axis (x) of the main body 11 of the device 10. The outer wall 16 extends at an angle outwardly from the longitudinal axis of the main body. The result is that the outer wall 16 forms a generally frusto-conical structure.

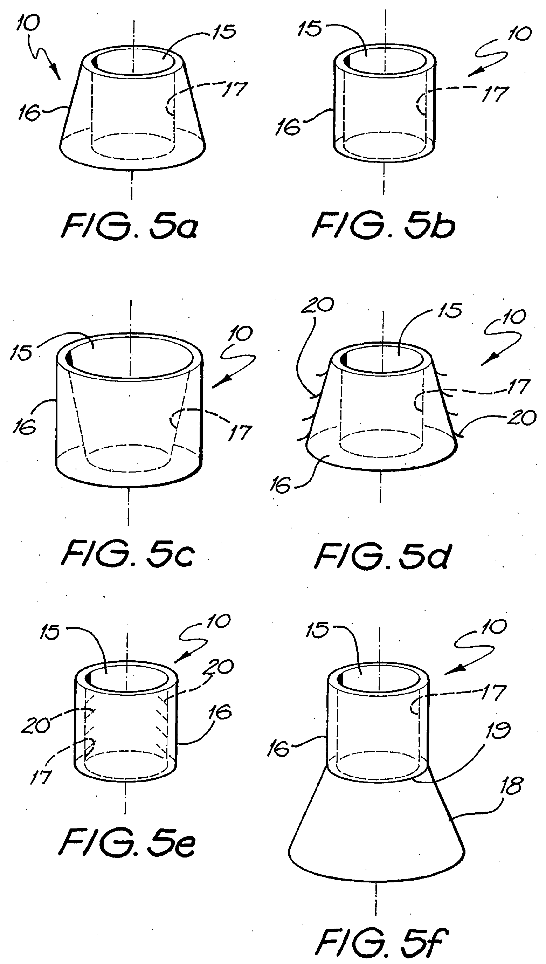

Because, the device of the present invention is designed to fit within vessels which have unique or unusual anatomical structures, the main body 11 may take on a number of configurations. For example, in Figure 5c, the internal wall 17 of the main body 11 may taper along the length of the main body 11.

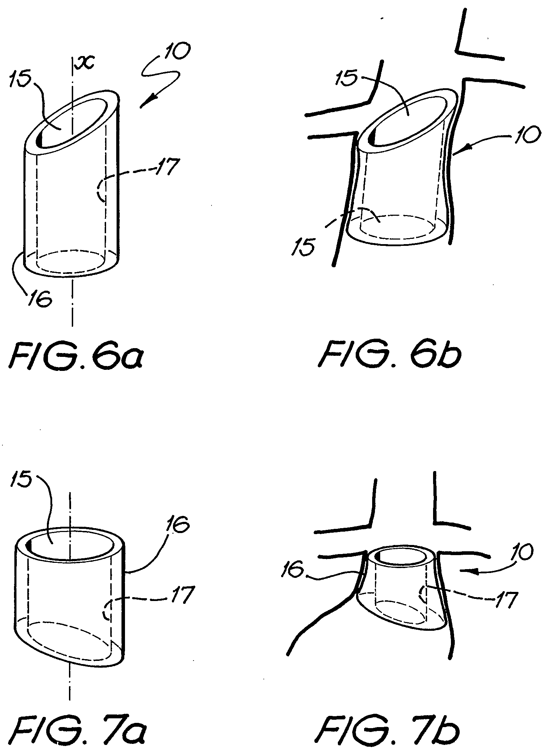

In figure 6a, and 7a, one end of the main body 11 is angled. This embodiment may be particularly useful in cases where the area of vessel to be engaged by the device and bridged by an intraluminal graft extends from a region adjacent two branching vessels. If the branching vessels are misaligned, however, a flat, rather than angled end may occlude one of the branching vessels (see Figure 6b). Similarly, if an aneurysm deviates from a vessel at a sharp angle, a main body having an angled end may be suitable for engaging the walls of this region (see Figure 7b).

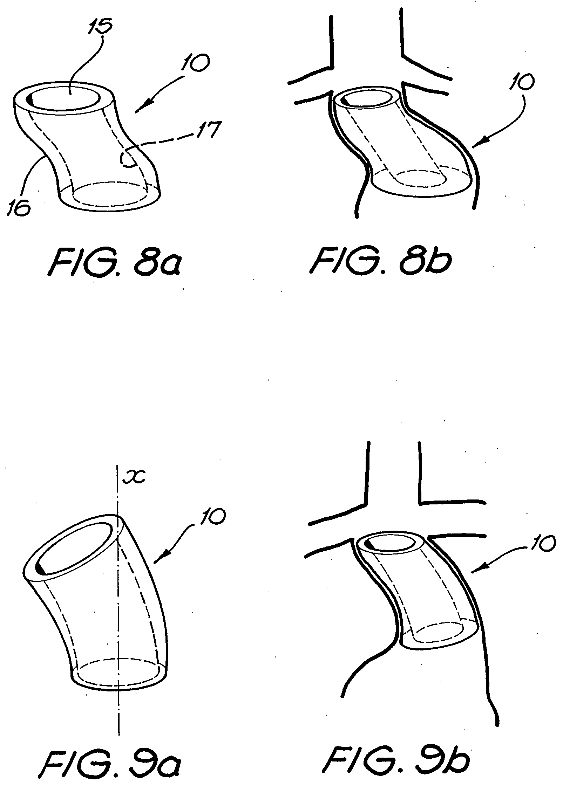

The entire main body 11 may be curved along its longitudinal axis (x) as depicted in Figure 9b.

In addition to comprising a main body 11, the device may further comprise a skirt member 18 which is attached a first end 19 of the main body 11 and depends therefrom.

The main body of the device may be made from any number of materials including polyurethane polymers including polyurethane polycarbonate. The main body may be a foam or sponge material. Different parts of the main body may be made from different types of material.

Preferably, the material of the main body is selected for its biocompatibility and ability to mould with the anatomy of the vessel in which it is positioned and to integrate with the tissue of the vessel wall. In this way, the main body of the device preferably becomes firmly integrated and secured within the vessel wall.

In Figures 5d and 5e, the outer wall 16 and the internal wall 17 respectively have projections 20 thereon. The projections 20 on the outer wall extend out and away from the main body 11. The projections 20 on the internal wall 17 of the main body 11 extend into the lumen 15 and are adapted to engage the intraluminal graft 13, securing the intraluminal graft 13 to the main body 11 of the device 10.

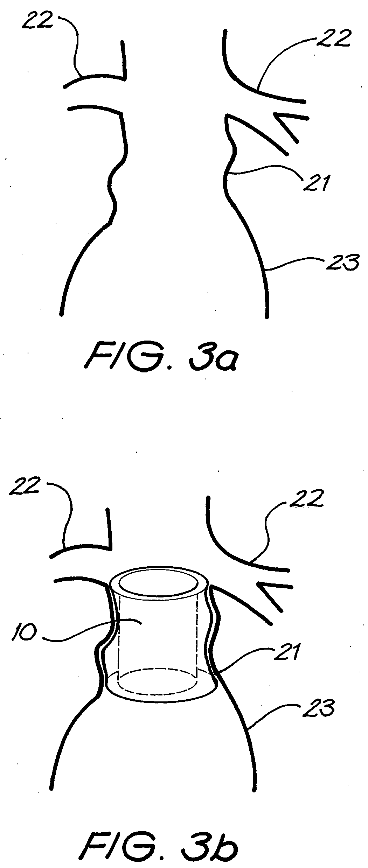

Figures 3a and 3b show a portion of the aorta 21 having a somewhat bulging, atypical appearance between the branch of the renal arteries 22 and an aneurysm 23. The device 10 is adapted to fit within the aorta 21 and engage the walls of the aorta 21 at the region immediately upstream of the aneurysm 23.

To introduce the device 10 into a target vessel such as the aorta 21, the device 10 is moved through an introducer catheter 24 in its radially compressed state (see Figure 2a). When the device reaches the target site, the introducer catheter 24 is withdrawn and the main body 11 caused or allowed to move to its radially expanded state (see Figure 2b) such that it engages with the vessel wall 14.

A proximal end 25 of the intraluminal graft 13 (in a radially compressed state) is then introduced into the lumen 15 and the intraluminal graft caused or allowed to move to a radially expanded state such that the wall of the graft 13 engages with the internal wall 17 of the main body 11. A region adjacent the proximal end 25 of the intraluminal graft 13 is therefore secured within the main body 11 and the remainder of the intraluminal graft 13 extends from the main body 11 and is free therefrom.

The introducer catheter may then be withdrawn.

The device 10 of the invention includes at least one stay 26 or holding catheter 27 releasably connected to a portion of the main body 11 of the device. The stay 26 or catheter 27 is preferably flexible but of sufficient rigidity to hold the device 10 in a desired position during deployment of the device 10 within a target vessel and during deployment of an intraluminal graft within said vessel.

The device 10 of the present invention and an intraluminal graft 13 may be packaged together as a single assembly (not shown) for delivery to a vessel of a patient. In this regard, the internal wall 17 of the main body of the device is attached to a portion of a wall of an intraluminal graft 13. Accordingly, the assembly may be carried through the introducer catheter 24 and released at the target site of a vessel. Upon release, the main body 11 of the device 10 is caused or allowed to move to its radially expanded state. The intraluminal graft 13 may be caused or allowed to radially expand upon release of the assembly or, alternatively, following the radial expansion of the main body 11 device 10.

The radial expansion of the intraluminal device may further fix the outer wall 16 of the main body 11 to a wall of the vessel 14, that is, an added force is applied to fix the device within the vessel.

It will be appreciated by persons skilled in the art that numerous variations and/or modifications may be made to the invention as shown in the specific embodiments without departing from the scope of the invention as broadly described. The present embodiments are, therefore, to be considered in all respects as illustrative and not restrictive.

## Claims

1. An engagement device (10) for an intraluminal graft or stent, the device comprising a main body (11) having (i) an inner wall which defines a receiving region to receive and engage at least a portion of an intraluminal graft or stent, and (ii) an outer wall (16) to engage the wall of a vessel of a patient, wherein said main body is capable of expanding from a first radially compressed state to a second radially expanded state such that when in its radially expanded state, the outer wall engages the wall of the vessel so that, in use, flow of blood in a vessel to which the device is fitted is channeled through the intraluminal graft or stent thereby preventing leakage of blood into the damaged part of the vessel;
and at least one stay (26) or holding catheter (27) releasably connected to a portion of the main body (11) of the device;
wherein, the at least one stay (26) or holding catheter (27) is adapted to hold in use the engagement device in a desired position during deployment of the device within the vessel and during deployment of the intraluminal graft or stent within said vessel.

2. The engagement device for an intraluminal graft or stent of claim 1 wherein the main body of the device is substantially cylindrical.

3. The engagement device for an intraluminal graft or stent of claim 1 or claim 2 wherein the main body of the device is generally frusto-conical in shape.

4. The engagement device for an intraluminal graft or stent of claim 3 wherein the internal wall is substantially straight relative to the longitudinal axis of the main body of the device and the outer wall is angled relative to the longitudinal axis of the main body.

5. The engagement device for an intraluminal graft or stent of any one of the preceding claims wherein the main body extends from a first end to a second end and wherein one or either end is angled relative to the longitudinal axis of the main body.

6. The engagement device for an intraluminal graft or stent of claim 1 wherein the main body of the device is curved relative to its longitudinal axis.

7. The engagement device for an intraluminal graft or stent of claim 1 wherein the main body of the device forms a partially sinusoidal configuration relative to its longitudinal axis.

8. The engagement device for an intraluminal graft or stent of any one of the preceding claims wherein the main body is made from a compressible foam or sponge material which is capable of self expanding from its first radially compressed state to its second radially expanded state in a vessel of a patient.

9. The engagement device for an intraluminal graft or stent of any one of claims 1 to 7 wherein the main body is caused to move from its first radially compressed state to its second radially expanded state by the force of an inflating balloon.

10. The engagement device for an intraluminal graft or stent of any one of the claims 1 to 7 wherein the main body of the device or a portion thereof is made from shape memory material wherein the patient's body temperature causes the temperature of the main body of the device to change, thereby enabling the main body to self-expand and take on a "memorised" shape.

11. The engagement device for an intraluminal graft or stent of any one of claims 1 to 7 wherein the main body of the device is spring expandable.

12. The engagement device of for an intraluminal graft or stent of any one of the preceding claims wherein the main body includes one or more projections (20) extending from its outer wall.

13. An intraluminal assembly for placement in a vessel of a patient, the assembly comprising:
(i) the engagement device (10) according to claim 1; and
(ii) an intraluminal graft or stent (13) capable of expanding or being expanded from a first radially compressed configuration to a second radially expanded configuration.

## Patentansprüche

1. Eingriffsvorrichtung (10) für einen intraluminalen Graft oder Stent, wobei die Vorrichtung einen Hauptkörper (11) umfasst, mit (i) einer Innenwand, die einen Aufnahmebereich für die Aufnahme und für den Eingriff mit mindestens einem Teil eines intraluminalen Graft oder Stent definiert; und (ii) einer Außenwand (16) für einen Eingriff mit der Wand eines Gefäßes eines Patienten, wobei sich der Hauptkörper aus einem ersten radial zusammengedrückten Zustand an einen zweiten radial ausgedehnten Zustand ausdehnen kann, so dass, wenn er sich in dessen radial ausgedehnten Zustand befindet, die Außenwand mit der Wand des Gefäßes eingreift, so dass im Einsatz die Blutströmung in einem Gefäß, an das die Vorrichtung angeschlossen ist, durch den intraluminalen Graft oder Stent geleitet wird, wodurch ein Austreten von Blut in den beschädigten Teil des Gefäßes verhindert wird;
und wenigstens eine Abstützung (26) oder einen Haltekatheter (27), die bzw. der lösbar mit einem Teil des Hauptkörpers (11) der Vorrichtung verbunden ist;
wobei die wenigstens eine Abstützung (26) oder der eine Haltekatheter (27) in der Lage ist, die Eingriffsvorrichtung im Einsatz während dem Einsatz der Vorrichtung in dem Gefäß und während dem Einsatz des intraluminalen Graft oder Stent in dem Gefäß an einer gewünschten Position zu halten.

2. Eingriffsvorrichtung für einen intraluminalen Graft oder Stent nach Anspruch 1, wobei der Hauptkörper der Vorrichtung im Wesentlichen zylindrisch ist.

3. Eingriffsvorrichtung für einen intraluminalen Graft oder Stent nach Anspruch 1 oder Anspruch 2, wobei der Hauptkörper der Vorrichtung allgemein kegelstumpfförmig ist.

4. Eingriffsvorrichtung für einen intraluminalen Graft oder Stent nach Anspruch 3, wobei die Innenwand im Wesentlichen gerade ist im Verhältnis zu der Längsachse des Hauptkörpers der Vorrichtung, und wobei die Außenwand angewinkelt ist im Verhältnis zu der Längsachse des Hauptkörpers.

5. Eingriffsvorrichtung für einen intraluminalen Graft oder Stent nach einem der vorstehenden Ansprüche, wobei sich der Hauptkörper von einem ersten Ende zu einem zweiten Ende erstreckt, und wobei ein Ende oder beide Enden angewinkelt ist bzw. sind im Verhältnis zu der Längsachse des Hauptkörpers.

6. Eingriffsvorrichtung für einen intraluminalen Graft oder Stent nach Anspruch 1, wobei der Hauptkörper der Vorrichtung gekrümmt ist im Verhältnis zu dessen Längsachse.

7. Eingriffsvorrichtung für einen intraluminalen Graft oder Stent nach Anspruch 1, wobei der Hauptkörper der Vorrichtung eine teilweise sinuskurvenförmige Konfiguration im Verhältnis zu dessen Längsachse bildet.

8. Eingriffsvorrichtung für einen intraluminalen Graft oder Stent nach einem der vorstehenden Ansprüche, wobei der Hauptkörper aus kompressiblem Schaumstoff oder Schwammmaterial besteht, der bzw. das sich in einem Gefäß eines Patienten selbst ausdehnen kann aus einem ersten radial zusammengedrückten Zustand an einen zweiten radial ausgedehnten Zustand.

9. Eingriffsvorrichtung für einen intraluminalen Graft oder Stent nach einem der Ansprüche 1 bis 7, wobei durch die Kraft eines sich aufblasenden Ballons bewirkt wird, dass sich der Hauptkörper aus dessen ersten radial zusammengedrückten Zustand an dessen zweiten radial ausgedehnten Zustand bewegt.

10. Eingriffsvorrichtung für einen intraluminalen Graft oder Stent nach einem der Ansprüche 1 bis 7, wobei der Hauptkörper der Vorrichtung oder ein Teil davon aus Formgedächtnismaterial besteht, wobei die Körpertemperatur des Patienten bewirkt, dass sich die Temperatur des Hauptkörpers der Vorrichtung verändert, wodurch es ermöglicht wird, dass sich der Hauptkörper selbst ausdehnt und eine "memorisierte" Form annimmt.

11. Eingriffsvorrichtung für einen intraluminalen Graft oder Stent nach einem der Ansprüche 1 bis 7, wobei der Hauptkörper der Vorrichtung federexpansionsfähig ist.

12. Eingriffsvorrichtung für einen intraluminalen Graft oder Stent nach einem der vorstehenden Ansprüche, wobei der Hauptkörper einen oder mehrere Vorsprünge (20) aufweist, die sich von dessen Außenwand erstrecken.

13. Intraluminale Einheit zur Platzierung in einem Gefäß eines Patienten, wobei die Einheit folgendes umfasst:
(i) die Eingriffsvorrichtung (10) nach Anspruch 1; und
(ii) einen intraluminalen Graft oder Stent (13), der sich ausdehnen kann oder der ausgedehnt werden kann aus einer ersten radial zusammengedrückten Konfiguration an eine zweite radial ausgedehnte Konfiguration.

## Revendications

1. Dispositif de mise en prise (10) pour une greffe ou endoprothèse intraluminale, le dispositif comprenant un corps principal (11) ayant (i) une paroi interne qui définit une région réceptrice pour recevoir et venir en prise avec au moins une partie d'une greffe ou endoprothèse intraluminale, et (ii) une paroi externe (16) pour venir en prise avec la paroi d'un vaisseau d'un patient, ledit corps principal pouvant se dilater d'un premier état radialement comprimé à un second état radialement dilaté de sorte que lorsqu'il se trouve dans son état radialement dilaté, la paroi externe vienne en prise avec la paroi du vaisseau de sorte que, lors de l'utilisation, le flux de sang dans un vaisseau sur lequel est posé le dispositif soit acheminé à travers la greffe ou endoprothèse intraluminale, empêchant ainsi les fuites de sang dans la partie endommagée du vaisseau ;
et au moins un appui (26) ou cathéter de maintien (27) raccordé amovible à une partie du corps principal (11) du dispositif ;
l'au moins un appui (26) ou cathéter de maintien (27) étant conçu pour maintenir lors de l'utilisation le dispositif de mise en prise dans une position voulue pendant le déploiement du dispositif dans le vaisseau et pendant le déploiement de la greffe ou endoprothèse intraluminale dans ledit vaisseau.

2. Dispositif de mise en prise pour une greffe ou endoprothèse intraluminale selon la revendication 1, le corps principal du dispositif étant sensiblement cylindrique.

3. Dispositif de mise en prise pour une greffe ou endoprothèse intraluminale selon la revendication 1 ou 2, le corps principal du dispositif étant d'une manière générale tronconique.

4. Dispositif de mise en prise pour une greffe ou endoprothèse intraluminale selon la revendication 3, la paroi interne étant sensiblement droite par rapport à l'axe longitudinal du corps principal du dispositif et la paroi externe étant inclinée par rapport à l'axe longitudinal du corps principal.

5. Dispositif de mise en prise pour une greffe ou endoprothèse intraluminale selon l'une quelconque des revendications précédentes, le corps principal s'étendant d'une première extrémité à une seconde extrémité et l'une ou l'autre extrémité étant inclinée par rapport à l'axe longitudinal du corps principal.

6. Dispositif de mise en prise pour une greffe ou endoprothèse intraluminale selon la revendication 1, le corps principal du dispositif étant courbé par rapport à son axe longitudinal.

7. Dispositif de mise en prise pour une greffe ou endoprothèse intraluminale selon la revendication 1, le corps principal du dispositif formant une configuration partiellement sinusoïdale par rapport à son axe longitudinal.

8. Dispositif de mise en prise pour une greffe ou endoprothèse intraluminale selon l'une quelconque des revendications précédentes, le corps principal étant en un matériau mousse ou éponge compressible pouvant se dilater automatiquement de son premier état radialement comprimé à son second état radialement dilaté dans un vaisseau d'un patient.

9. Dispositif de mise en prise pour une greffe ou endoprothèse intraluminale selon l'une quelconque des revendications 1 à 7, le corps principal est amené à passer de son premier état radialement comprimé à son second état radialement dilaté par la force d'un ballonnet gonflé.

10. Dispositif de mise en prise pour une greffe ou endoprothèse intraluminale selon l'une quelconque des revendications 1 à 7, le corps principal du dispositif ou une partie de celui-ci étant en un matériau à mémoire de forme, la température corporelle du patient amenant la température du corps principal du dispositif à changer, permettant ainsi au corps principal de se dilater automatiquement et de prendre une forme « mémorisée ».

11. Dispositif de mise en prise pour une greffe ou endoprothèse intraluminale selon l'une quelconque des revendications 1 à 7, le corps principal du dispositif pouvant être dilaté par ressort.

12. Dispositif de mise en prise pour une greffe ou endoprothèse intraluminale selon l'une des revendications précédentes, le corps principal comprenant une ou plusieurs saillies (20) s'étendant de sa paroi externe.

13. Ensemble intraluminal à placer dans un vaisseau d'un patient, l'ensemble comprenant :
(i) le dispositif de mise en prise (10) selon la revendication 1 ; et
(ii) une greffe ou endoprothèse intraluminale (13) pouvant se dilater ou étant dilatée à partir d'une première configuration radialement comprimée vers une seconde configuration radialement dilatée.
